# EUROPEAN PATENT SPECIFICATION

(11) **EP 1 490 144 B1**
(45) Date of publication and mention of the grant of the patent: **08.08.2007**
(21) Application number: 03706344.3
(22) Date of filing: 11.03.2003
(51) Int. Cl.: A61M 37/00, A61M 5/00

(54) **APPARATUS FOR AUTOMATIC INSERTION OF A SOLID MEDICINE**
VORRICHTUNG ZUM AUTOMATISCHEN EINSETZEN EINES FESTEN ARZNEIMITTELS
APPAREIL POUR L'INSERTION AUTOMATIQUE D'UN MEDICAMENT SOUS FORME SOLIDE

(30) Priority: 22.03.2002 DK 200200444
(43) Date of publication of application: 29.12.2004
(73) Proprietor: NOVO NORDISK A/S, 2880 Bagsvaerd (DK)
(72) Inventor: SABRA, Mads, Christian, DK-2200 Copenhagen N (DK)
(86) International application number: PCT/DK2003/000149
(87) International publication number: WO 2003/080173

(56) References cited:
- WO-A-01/68168

## Description

The invention relates to an apparatus for automatic insertion of medicine pegs into the skin.

Some medicaments are injected as heavy soluble crystals or particles suspended in a solvent. When injected the heavy soluble medicine is during time dissolved by the tissue liquid. Instead of suspending or dissolving the medicine in a solvent the dry medicine may be pressed to a small needle shaped peg which can be inserted into the tissue from where it is during time dissolved by the tissue liquid.

From US 5542920 is known a device by which a peg stored in a barrel which is provided in a housing and from which said peg can be pressed into the skin by a plunger which can be passed into the barrel by pressing a button. Alternatively the peg can be shot into the skin by a spring or by applying a gas pressure behind the peg in the barrel.

When the peg is pressed into the skin by a plunger which is pressed into the barrel from one end thereof whereas the other end of the barrel is pressed against the skin where the insertion is wanted, the plunger must be pressed to a position where its end facing the peg is passed some millimetres into the skin to ensure that the peg is inserted to a subcutaneous position. Thereafter it is wanted to have the plunger drawn back into the barrel so that the end of the plunger, which may have been contaminated by the insertion, is hidden in the barrel. This may be obtained by providing a spring between the housing and the button by which the plunger is pressed into the barrel. By the insertion movement of the button this spring is compressed and when the button is released said spring will press the button back and this way draw the plunger back from its peg insertion position leaving the peg subcutaneously inserted.

It is an objective of the invention to provide a small handy device by which a solid medicine peg may be automatically inserted.

This is obtained by an apparatus for automatic insertion of medicine pegs into the skin which apparatus has a barrel in which a medicine peg is stored and in which a plunger behind said peg can be moved to press the peg out through an end of the barrel which end is pressed against the skin on the location where the peg is intended to be inserted, which apparatus is according to the invention characterised in that the plunger is a part of a flexible rod construction which is so designed that it can be passed from a first non rectilinear constellation, by which the plunger is withdrawn in the barrel to a position leaving space for the medicine peg, to a rectilinear constellation by which the plunger projects from the end of the barrel.

According to the invention the rod constellation may be a single flexible bar having a first and a second end, said first end being fixed in the housing an said second end being placed behind a medicine peg in the barrel, which flexible bar between said first and second ends has a deflection in a first direction perpendicular to its axis, spring mechanisms being provided which act on the deflection in a direction to straighten the deflection and provide a deflection in a second direction opposite said first direction.

The spring mechanism may be provided by an inherent spring force in the flexible bar which is formed with a deflection in said second direction and against its inherent spring force is forced to have a deflection in said first direction.

The bar is held in its position with a deflection in said first direction until a release mechanism is activated to set free the spring forces which will force the bar over a rectilinear position to a position with a deflection in said second direction. When the bar is moving towards its rectilinear position, the end of the bar which forms a plunger will press the medicine peg out of the barrel and into the skin when the outer end of the barrel is pressed against the skin. When the bar reaches its rectilinear position, the plunger projects a short distance from the end of the barrel whereby it is ensured that the peg is pressed to a position the same distance under the skin surface. By the further movement of the bar to its position with a deflection in the second direction, the end of the plunger is withdrawn from the skin so that the device can be removed from its contact with the skin without any risk of scratching.

In another embodiment the spring mechanism may comprise a spring provided and compressed between the housing and the flexible rod when this rod is in its first non rectilinear position, so that the spring presses the flexible rod towards its rectilinear position.

In another embodiment the flexible rod constellation may be formed by three rectilinear rods which are through links coupled in series so that one rod forms the plunger whereas a knee link mechanism is formed by a first rod linked between the plunger and a second rod, which is linked between the first rod and a point in the housing which point lies on the extension of the plunger rod axis. A spring act on the knee link mechanism where the first rod is coupled to the second rod in a direction so that a straightening of the knee link is attempted. When the knee link is straightened the peg is pressed out of the barrel and the plunger project a short distance from the end of the barrel. When the further action of the spring moves the knee link mechanism to a new bent position the plunger is drawn back into the barrel.

During the movement from its initial position to its final position the second rod rotates about a pin fixed in the housing. It may be attractive to provide a fly wheel which can help said second rod in its rotation towards its final position. This can be obtained by replacing the second rod by a disk rotating about said pin fixed in the housing. The rotation of the disk can be supported by a spring acting on the disk to provide a torque about said pin.

The rod constellation described has the advantage that it provides in a very simple way a gearing transforming a movement provided by a small force, the movement of the deflection provided by the spring mechanism, to a shorter movement with a stronger force, the movement of the plunger which presses the peg into the skin.

### In the following the invention is further described with references to the drawing, wherein

- Figure 1: shows schematically a device according to the invention with its rod constellation in its initial non rectilinear position,
- Figure 2: shows the device in figure with its rod constellation in its rectilinear position,
- Figure 3: shows the device of figure 1 and 2 with its rod constellation in its final non rectilinear position,
- Figure 4: shows schematically another embodiment of a device according to the invention wherein the rod constellation is represented by one integral flexible rod in an initial non rectilinear position,
- Figure 5: shows the device of figure 4 with the rod in its rectilinear position, and
- Figure 6: shows the device of figure 4 and 5 with the rod in its final non rectilinear position.

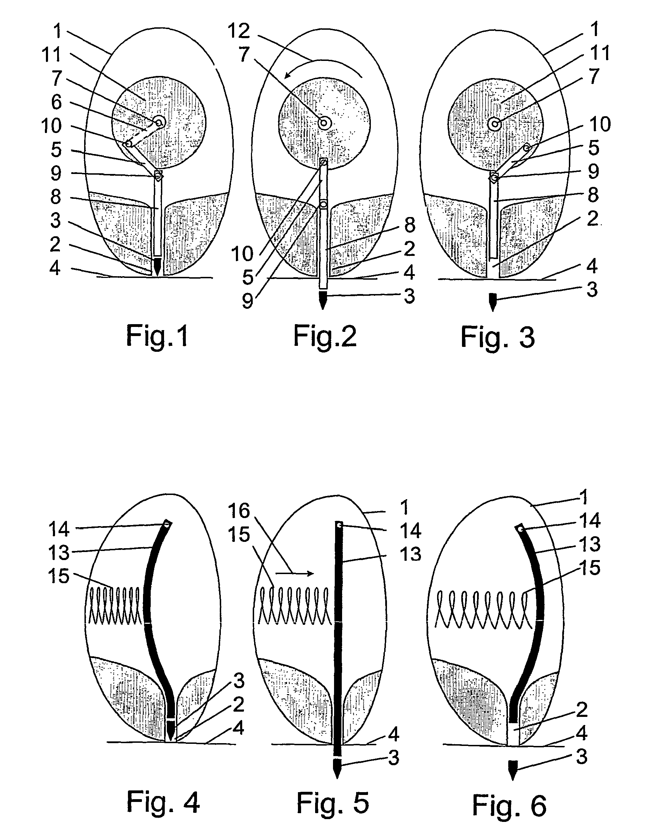

In figure 1 is a housing 1 provided with a barrel 2 in which a peg 3 of solid medicine is placed at the outlet of the barrel 2 ready to be passed through the skin 4 of a patient. In the barrel 2 behind the peg 3 a rod shaped plunger 8 is placed which plunger by a first (5) and a second rod (6) rod is connected to a pivot pin 7 in the housing 1. The first rod 5 is at its one end coupled to an inner end of the plunger by a link 9 and at its other end coupled to the second rod 6 by a link 10. The lengths of the plunger 8 and the rods 5 and 6 are so adjusted that they form a knee link mechanism between the pin 7 and the inner end of the plunger 8 when said plunger is in its position behind a peg 3 in the barrel 2. The second rod 6 can be rotated about the pivot pin 7 and this rotation can be supported by a flywheel 11 into which the second rod can be integrated as shown in figure 2 and 3.

In figure 2 the flywheel 11 has been rotated so far in the direction indicated by the arrow 12 that the plunger 8, the links 9 and 10, and the pivot pin 7 lies on one straight line, i.e. the rods of the knee link mechanism and the plunger are brought to a rectilinear position. The lengths of the plunger and the first and the second rods are so adjusted that the plunger in this position projects from the outer end of the barrel a short distance. Thereby it is ensured that the peg 3 is positioned a short distance beneath the surface of the skin 4. The distance is set by the choice of the lengths of the rods 5 and 6 and the plunger 8. The length of the integrated second rod is defined by the distance between the pivot pin 7 and the link 10.

In figure 3 the flywheel 11 and its integrated second rod has been further rotated to a position wherein the plunger 8 and the first and the second rod does no longer attain a rectilinear constellation. Thereby the plunger 8 is withdrawn from its position projecting from the barrel 2 and leaves the peg 3 a suitable distance beneath the skin 4.

The device can be a disposable device wherein the rotation of the second rod 6 and the flywheel is induced by a not shown spring working between the housing and the flywheel 11 or the second rod 6. When the device is stored it will be in the position shown in figure 1 with the spring cocked. When the apparatus is going to be used the opening of the barrel is pressed against the skin and the cocked spring is released. Thereby the second rod is rotated in the direction shown by the arrow 12 in figure 2. The rod is rotated through the position shown in figure 2 to the position shown in figure 3 where the plunger is drawn back into the barrel. Hidden in the barrel the plunger, which may be contaminated by the perforation of the skin, cannot get in touch with anybody.

Figure 4, 5, and 6 shown another embodiment of a device according to the invention. In this embodiment the plunger rod and the first and second rod of the knee link mechanism is replaced by one single flexible rod 13 which is at one end guided in the barrel 2 and is at the other end rotatably fixed in the housing by a pin 14. The flexible rod can be a rectilinear rod which is in figure 1 held in a bent out position in which the free end of the rod 13 is positioned a distance from the mouth of the barrel 2 allowing a peg to be placed in this barrel adjacent to the mouth thereof.

When the rod 13 is released it will pass to a rectilinear position either by its own inherit spring force or by the force of a spring 15 acting on the rod perpendicular to its axis between the free end and the end fixed in the housing 1. From its rectilinear position the flexible rod will further move in the direction shown by an arrow 16 in figure 5 to a new bent out position shown in figure 6 in which said rod is withdrawn from its projecting position.

## Claims

1. An apparatus for automatic insertion of a medicine peg (3) into the skin, which apparatus has a barrel (2) wherein a medicine peg (3) is stored and in which a plunger (8) behind said peg can be moved to press the peg (3) out through an end of the barrel (2), which end is adapted to be pressed against the skin on the location where it is intended to insert the peg (3), **characterised in that** the plunger (8) is a part of a flexible rod construction (5, 6, 7, 9, 10) which is so designed that it can be passed from a first non rectilinear constellation, by which the plunger (8) is withdrawn in the barrel (2) to a position leaving space for the medicine peg (3), to a rectilinear constellation by which the plunger (8) projects from the end of the barrel (2).

2. An apparatus according to claim 1, **characterised in that** a spring (15) is provided and cocked between the housing (1) and the flexible rod construction (5, 6, 7, 9, 10) when this rod construction occupies its first non rectilinear constellation, so that the spring (15) presses the flexible rod construction towards its rectilinear constellation.

3. An apparatus according to claim 2, **characterised in that** the spring (15) is so dimensioned that l can pres the flexile rod construction (5, 6, 7, 9, 10) past its rectilinear constellation to a second non rectilinear constellation.

4. An apparatus according to claim 1, **characterised in that** the flexible rod construction (5, 6, 7, 9, 10) is formed by three rectilinear rods (5, 6, 8) which are through links (7, 9, 10) coupled in series so that one rod forms the plunger (8) whereas a knee link mechanism (9) is formed by a first rod (5) linked between the plunger (8) and a second rod (6), which is linked between said first rod (5) and a pin (7) in the housing which pin lies on the extension of the plunger rod axis.

5. An apparatus according to claim 4 **characterised in that** a spring (15) is provided which is cocked so that it act on the second rod (6) wich is linked to the housing to rotate this second rod (6) from a position in which the first and second rod and the plunger forms a non rectilinear constellation, through a position in which such an rectilinear constellation is formed, and further to a second non rectilinear constellation.

6. An apparatus according to claim 4 or 5, **characterised in that** a flywheel (11) rotating about said pin (7) in the housing (1) is provided coupled to the second rod (6) to support the rotation of this rod about the pin (7).

7. An apparatus according to claim 6, **characterised in that** the second rod (6) is integrated in the flywheel (11) to which the first rod (5) is linked in a point in a distance from said pin (7).

## Patentansprüche

1. Vorrichtung zum automatischen Einführen eines Medikamentenzapfens (3) in die Haut, wobei die Vorrichtung einen Zylinder (2) umfasst, in welchem ein Medikamentenzapfen (3) aufbewahrt wird und in welchem ein Kolben (8) hinter den Zapfen bewegt werden kann, um den Zapfen (3) durch ein Ende des Zylinders (2) herauszudrücken, wobei das Ende dazu angepasst ist, an der Stelle, an welcher es vorgesehen ist, den Zapfen (3) einzuführen, gegen die Haut zu drücken; **dadurch gekennzeichnet, dass** der Kolben (8) ein Teil einer beweglichen Stangenkonstruktion (5, 6, 7, 9, 10) ist, die derart gestaltet ist, dass sie von einer ersten nicht geradlinigen Konstellation, durch welche der Kolben (8) in den Zylinder (2) zu einer Position, die einen Raum für den Medikamentenzapfen (3) hinterlässt, eingefahren wird, zu einer geradlinigen Konstellation, durch welche der Kolben (8) aus dem Ende des Zylinders (2) herausragt, geführt werden kann.

2. Vorrichtung nach Anspruch 1, **dadurch gekennzeichnet, dass** eine Feder (15) bereitgestellt und zwischen dem Gehäuse (1) und der beweglichen Stangenkonstruktion (5, 6, 7, 9, 10) gespannt ist, wenn diese Stangenkonstruktion ihre erste nicht geradlinige Konstellation belegt, sodass die Feder (15) die bewegliche Stangenkonstruktion zu ihrer geradlinigen Konstellation drückt.

3. Vorrichtung nach Anspruch 2, **dadurch gekennzeichnet, dass** die Feder (15) derart bemessen ist, dass sie die bewegliche Stangenkonstruktion (5, 6, 7, 9, 10) nach ihrer geradlinigen Konstellation zu einer zweiten nicht geradlinigen Konstellation drücken kann.

4. Vorrichtung nach Anspruch 1, **dadurch gekennzeichnet, dass** die bewegliche Stangenkonstruktion (5, 6, 7, 9, 10) durch drei geradlinige Stangen (5, 6, 8) gebildet ist, die durch Verbindungsglieder (7, 9, 10) in Reihe gekoppelt sind, so dass eine Stange den Kolben (8) bildet, während ein Knieverbindungsmechanismus (9) durch eine erste Stange (5), die zwischen dem Zylinder (8) eingebunden ist, und eine zweite Stange (6), die zwischen der ersten Stange (5) und einem Stift (7) im Gehäuse eingebunden ist, gebildet ist, wobei der Stift an der Verlängerung der Kolbenstangenachse liegt.

5. Vorrichtung nach Anspruch 4, **dadurch gekennzeichnet, dass** eine Feder (15) bereitgestellt ist, die derart gespannt ist, dass sie auf die zweite Stange (6), die an das Gehäuse gebunden ist, einwirkt, um diese zweite Stange (6) von einer Position, in welcher die erste und die zweite Stange und der Kolben eine nicht geradlinige Konstellation bilden, durch eine Position, in welcher eine derartige geradlinige Konstellation gebildet wird, und weiter zu einer zweiten nicht geradlinigen Konstellation zu drehen.

6. Vorrichtung nach Anspruch 4 oder 5, **dadurch gekennzeichnet, dass** ein Schwungrad (11), dass sich um den Stift (7) im Gehäuse (1) dreht, derart bereitgestellt ist, dass es an die zweite Stange (6) gekoppelt ist, um die Drehung dieser Stange um den Stift (7) zu unterstützen.

7. Vorrichtung nach Anspruch 6, **dadurch gekennzeichnet, dass** die zweite Stange (6) in das Schwungrad (11) integriert ist, an welches die erste Stange (5) in einen Punkt mit einem Abstand von dem Stift (7) eingebunden ist.

## Revendications

1. Appareil destiné à l'introduction automatique d'un médicament de forme pointue (3) dans la peau, lequel appareil comprend une partie cylindrique (2) dans laquelle un médicament de forme pointue (3) est conservé, et dans laquelle un plongeur (8), placé derrière ledit médicament de forme pointue, peut être déplacé afin d'expulser le médicament de forme pointue (3) par une extrémité de la partie cylindrique (2), laquelle extrémité est propre à être pressée contre la peau à l'emplacement où il est prévu d'introduire le médicament de forme pointue (3), **caractérisé en ce que** le plongeur (8) fait partie d'une structure de tiges flexibles (5, 6, 7, 9, 10) qui est conçue de manière à pouvoir passer d'une première disposition selon une constellation non rectiligne, dans laquelle le plongeur (8) est ramené à l'intérieur de la partie cylindrique (2) jusqu'à une position laissant de l'espace pour le médicament de forme pointue (3), à une disposition selon une constellation rectiligne dans laquelle le plongeur (8) dépasse de l'extrémité de la partie cylindrique (2).

2. Appareil selon la revendication 1, **caractérisé en ce qu'**un ressort (15) est prévu et serré entre le boîtier (1) et la structure de tiges flexibles (5, 6, 7, 9, 10) lorsque cette structure de tiges est dans sa première disposition selon une constellation non rectiligne, si bien que le ressort (15) exerce une pression sur la structure de tiges flexibles pour qu'elle prenne sa disposition selon une constellation rectiligne.

3. Appareil selon la revendication 2, **caractérisé en ce que** le ressort (15) est dimensionné de manière à pouvoir pousser la structure de tiges flexibles (5, 6, 7, 9, 10) au-delà de sa disposition selon une constellation rectiligne, vers une seconde disposition selon une constellation non rectiligne.

4. Appareil selon la revendication 1, **caractérisé en ce que** la structure de tiges flexibles (5, 6, 7, 9, 10) est formée par trois tiges rectilignes (5, 6, 8) qui sont par des biellettes (7, 9, 10) montées en série, de telle sorte qu'une tige constitue le plongeur (8) tandis qu'un mécanisme d'articulation coudée (9) est formé par une première tige (5) articulée entre le plongeur (8) et une deuxième tige (6), qui est articulée entre ladite première tige (5) et un axe de pivotement (7) situé dans le boîtier, lequel axe est situé sur le prolongement de l'axe de la tige formant plongeur.

5. Appareil selon la revendication 4, **caractérisé en ce qu'**un ressort (15) est prévu et serré de manière à agir sur la deuxième tige (6), à montage de manière articulée sur le boîtier afin de faire tourner cette deuxième tige (6) depuis une position dans laquelle les première et deuxième tiges et le plongeur forment une disposition selon une constellation non rectiligne, en passant par une position dans laquelle une disposition selon une constellation rectiligne est obtenue, et au-delà jusqu'à une seconde disposition selon une constellation non rectiligne.

6. Appareil selon la revendication 4 ou 5, **caractérisé en ce qu'**il est prévu un volant (11) tournant autour dudit axe de pivotement (7) dans le boîtier (1), et relié à la deuxième tige (6) pour aider à la rotation de cette tige autour de l'axe (7).

7. Appareil selon la revendication 6, **caractérisé en ce que** la deuxième tige (6) est intégrée au volant (11), la première tige (5) y étant reliée de façon articulée en un point situé à une certaine distance dudit axe de pivotement (7).
